# FASCICULE DE BREVET EUROPEEN

(11) **EP 0 673 625 B1**
(45) Date de publication et mention de la délivrance du brevet: **12.05.1999**
(21) Numéro de dépôt: 95400618.5
(22) Date de dépôt: 21.03.1995
(51) Int. Cl.: A61B 17/06

(54) **Conditionnement pour suture chirurgicale**
Verpackung für chirurgisches Nahtmaterial
Package for surgical suture

(30) Priorité: 22.03.1994 FR 9403337
(43) Date de publication de la demande: 27.09.1995
(73) Titulaire: ETHICON, INC., Somerville, New Jersey 08850 (US); ETHNOR, F-92200 Neuilly sur Seine (FR)
(72) Inventeur: PERNOT, Pascal Cyrille Roland, F-28000 CHARTRES (FR)
(74) Mandataire: Texier, Christian

(56) Documents cités:
- EP-A- 0 046 518
- EP-A- 0 115 580
- EP-A- 0 393 326
- DE-A- 2 532 992
- US-A- 4 034 850
- US-A- 4 063 638
- US-A- 4 120 395
- US-A- 4 555 016
- US-A- 4 572 363
- US-A- 4 884 681
- US-A- 5 101 968

## Description

La présente invention concerne le domaine des conditionnements de suture chirurgicale.

De nombreux conditionnements pour suture chirurgicale ont déjà été proposés.

On a proposé par exemple des conditionnements comprenant des berceaux en matière plastique conçus pour être placés dans un fourreau en carton avant d'être introduits dans une enveloppe externe stérile.

On a également proposé des conditionnements pour suture chirurgicale comprenant de simples emballages cartonnés destinés à être placés dans une enveloppe externe stérile.

Le but de la présente invention est de perfectionner les conditionnements de suture chirurgicale antérieurs connus.

Un but important de la présente invention est en particulier de proposer un nouveau conditionnement pour suture chirurgicale conçu pour faciliter l'accès à l'aiguille de la suture.

Un emballage support de suture chirurgicale selon le préambule de la revendication 1 est connu de US-A-4 063 638.

Ce but est atteint dans le cadre de la présente invention grâce à un emballage-support de suture chirurgicale, caractérisé en ce qu'il est réalisé par découpes et pliages d'un flanc en carton comprenant trois éléments séparés deux à deux par des lignes de pliage respectives, le fil de la suture étant situé essentiellement entre deux éléments adjacents de l'emballage support et l'emballage comprenant un volet d'ouverture articulé à pivotement sur le troisième élément et l'aiguille de la suture étant fixée sur ce volet d'ouverture à distance de son axe d'articulation, et de la zone d'émergence du fil entre les deux éléments premiers cités, de sorte que lors de l'ouverture de l'emballage support, le pivotement du volet d'ouverture provoque le décollement de l'aiguille en éloignement de la structure de base de l'emballage support et facilite de ce fait la préhension ultérieure de l'aiguille.

Selon une autre caractéristique avantageuse de l'invention, les moyens de fixation de l'aiguille sur le volet d'ouverture sont formés d'un bloc de mousse adapté de sorte que lors du pivotement du volet d'ouverture, non seulement l'aiguille s'éloigne de la structure de base de l'emballage support, mais également pivote à l'intérieur du bloc de mousse. Ce pivotement permet à l'aiguille de se retrouver décollée du volet d'ouverture permettant une préhension adéquate.

D'autres caractéristiques, buts et avantages de la présente invention apparaîtront à la lecture de la description détaillée qui va suivre, et en regard des dessins annexés donnés à titre d'exemple non limitatif et sur lesquels :
- la figure 1 représente une vue schématique en plan d'un conditionnement de suture chirurgicale conforme à la présente invention,
- la figure 2 représente une vue en plan de l'emballage support conforme à la présente invention, après ouverture du volet articulé à pivotement,
- la figure 3 représente une vue en perspective de même emballage support, après ouverture du volet articulé à pivotement précité, et
- la figure 4 représente une vue en plan du flanc cartonné utilisé pour la réalisation d'un emballage support conforme à la présente invention.

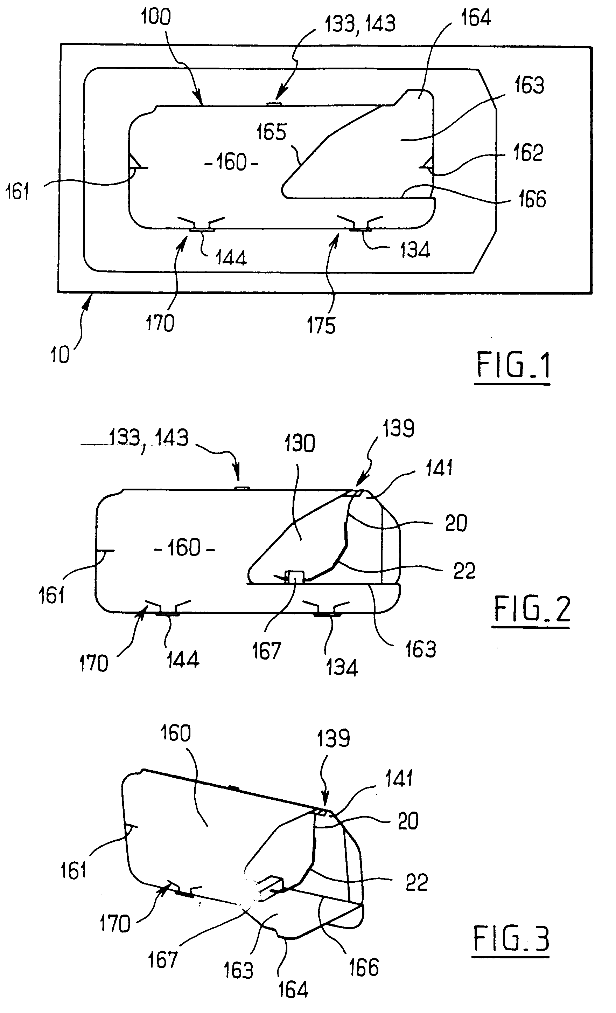

Comme on le voit sur la figure 1 annexée, le conditionnement pour suture chirurgicale conforme à la présente invention comprend essentiellement un emballage-support 100 placé dans une enveloppe externe 10.

L'enveloppe externe 10 est classique en elle-même et ne sera donc pas décrite dans le détail par la suite.

On rappelle cependant que cette enveloppe externe 10 est stérile. Elle peut être formée avantageusement de deux feuilles pelables reliées sur leurs périphéries pour former ainsi une poche interne fermée apte à contenir l'emballage support 10. A titre d'exemple non limitatif, l'enveloppe externe 10 peut être formée d'une feuille de base papier et d'une seconde feuille en matière plastique optiquement transparente.

Sur les dessins annexés, le fil de suture est référencé 20, tandis que l'aiguille est référencée 22.

On va maintenant préciser la structure de l'emballage support 100 conforme à la présente invention.

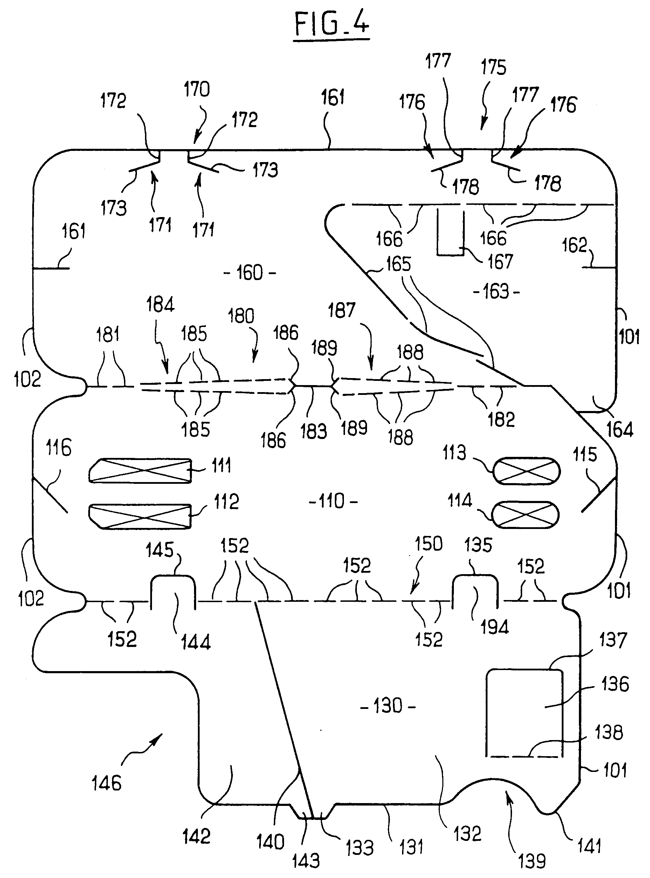

Cet emballage support 100 est réalisé par découpe et pliage d'un flanc cartonné unique visible sur la figure 4.

Comme on le voit à l'examen de cette figure 4, le flanc cartonné présente un contour initial globalement rectangulaire. Plus précisément, on délimite par pliage, dans ce flanc, trois éléments 110, 130 et 160. Ces trois éléments 110, 130 et 160 sont séparés deux à deux par des lignes d'articulation 150, 180 qui seront décrites plus en détail par la suite.

Pour simplifier la description détaillée qui va suivre, on appellera ultérieurement l'élément 110 : l'élément médian, l'élément 130 : l'élément interne (du fait que celui-ci est placé à l'intérieur de l'emballage après fermeture de ce dernier), et l'élément 160 : l'élément externe (du fait que celui-ci est placé sur l'extérieur de l'emballage après fermeture de ce dernier).

De plus, on appellera bord longitudinal interne et bord longitudinal externe respectivement les bords du flanc référencés 131, 161 sur la figure 4 et bords latéraux les bords référencés 101, 102 sur la figure 4. Les bords latéraux 101, 102 sont orthogonaux aux bords longitudinaux 131, 161.

Les lignes de pliage 150, 180 précitées s'étendent globalement parallèlement aux bords longitudinaux 131, 161 et perpendiculairement aux bords latéraux 101,102.

La figure 4 correspond à la face intérieure du flanc ou emballage.

La ligne de pliage 150 qui sépare l'élément médian 110, de l'élément interne 130, est formée d'une série de découpes 152 constituées de segments rectilignes non jointifs parallèles aux bords longitudinaux 131,161.

Les segments 150, 152 s'étendent ainsi globalement entre les bords latéraux 101, 102.

Ces segments 152 sont tous alignés.

La seconde ligne de pliage 180 est formée entre l'élément médian 110 et l'élément externe 160.

Cette ligne de pliage 180 est constituée de :
- deux segments de découpe 181 rectilignes non jointifs et alignés adjacents au bord latéral 102,
- deux segments 182 de découpe rectilignes non jointifs alignés entre eux et alignés sur les segments 181, adjacents au bord latéral 101,
- un segment de découpe central 183 rectiligne et aligné sur les segments 181, 182 précités, et
- deux ensembles symétriques de découpes 184, 187 situés respectivement l'un entre les segments 181 et le segment central 183, l'autre entre les segments 182 et le segment central 183.

Chacun des deux ensembles 184, 187 comprend deux séries de découpes 185, 188 respectivement. Chaque série de découpes 185, 188 est formée de segments rectilignes alignés. Par ailleurs les deux séries de découpes respectivement 185 et 188 de chaque ensemble 184, 187 divergent en rapprochement du segment central 183.

En outre, chacun des ensembles 184, 187 comprend deux segments additionnels 186, 189 respectivement en "V". Ainsi, les segments additionnels 186, 189 convergent en rapprochement du segment central 183 et relient respectivement les extrémités de ce segment central 183 aux extrémités des séries de segments 185, 188 précités.

La ligne de pliage 180 ainsi formée par les segments de découpe 181 à 189 a pour but de donner du volume à l'emballage-support 100. L'homme de l'art comprendra en effet aisément qu'après pliage l'épaisseur de l'emballage-support 100 correspond à l'écartement entre les deux séries de segments de découpe 185 et 188 respectivement. En d'autres termes, I'épaisseur de l'emballage-support croît progressivement, à partir des bords latéraux 101, 102 vers le centre de l'emballage.

On va maintenant préciser la structure de l'élément médian 110.

Cet élément médian 110 comprend deux paires de fenêtres 111, 112, 113, 114 traversantes. Chaque fenêtre 111, 112, 113, 114 présente un contour globalement rectangulaire allongé à coins arrondis. Chaque paire de fenêtres 111, 112 et 113, 114 respectivement présente une symétrie par rapport à un plan médian orthogonal à l'élément 110 et parallèle aux lignes de pliage 150, 180.

La première paire de fenêtres 111, 112 est située à proximité du bord latéral 102 tandis que la seconde paire de fenêtres 113, 114 est située à proximité du second bord latéral 101. Les fenêtres 111, 112, 113 et 114 sont allongées parallèlement aux lignes de pliage 150, 180.

Le but des fenêtres 111, 112, 113 et 114 est d'autoriser le passage de broches servant, de façon classique en soi, de supports provisoires aux fils de suture, au cours du bobinage de celui-ci.

L'élément médian 110 comprend en outre, sur ses bords latéraux 101, 102 respectivement, une découpe rectiligne 115, 116.

Les découpes 115, 116 sont inclinées par rapport aux bords latéraux 101, 102 avantageusement de 45°.

Les découpes 115, 116 convergent vers la ligne de pliage 150 en direction de l'intérieur du flanc cartonné 100.

Les découpes 115, 116 débouchent respectivement sur les bords latéraux 101, 102 sensiblement à mi-longueur de ceux-ci sur l'élément médian 110.

Plus précisément, le point milieu des découpes 115, 116 est de préférence situé au droit du milieu des bords latéraux 101, 102 de l'élément médian 110, de sorte que les découpes 115, 116 débouchent entre ce point milieu des bords latéraux 101, 102 et la ligne de pliage 180.

On va maintenant décrire la structure de l'élément interne 130.

Cet élément interne 130 est avantageusement scindé en deux parties 132, 142 par une découpe 140.

La découpe 140 est avantageusement rectiligne. Elle relie la ligne de pliage 150 au bord longitudinal 131. Plus précisément encore, la ligne de découpe rectiligne 140 est avantageusement inclinée par rapport à la ligne de pliage 150 et au bord longitudinal 131, c'est-à-dire qu'elle n'est pas perpendiculaire à ceux-ci.

La découpe 140 converge vers le bord latéral 101 en rapprochement du bord longitudinal 131.

La découpe 140 débouche sur le bord longitudinal 131 sensiblement à mi-longueur du bord longitudinal 131.

L'élément interne 130 comprend deux saillies 133, 143 qui s'étendent au-delà du bord longitudinal 131, respectivement de part et d'autre de la découpe 140, soit respectivement sur les deux parties 132, 142.

La largeur de l'élément interne 130 considérée parallèlement aux bords latéraux 101, 102 étant égale à la largeur correspondante de l'élément médian 110, ces deux saillies 133, 143 ont pour but de pénétrer dans la découpe centrale 183 précitée pour permettre de maintenir en position refermée accollée, l'élément interne 130 et l'élément médian 110.

Au niveau de la ligne de pliage 150, les deux parties 132, 142 de l'élément interne 130 sont munies chacune d'une oreille en saillie 134, 144. Ces oreilles 134, 144 ont de préférence un contour rectangulaire. Elles sont délimitées par une découpe en forme de U 135, 145. Ces oreilles 134, 144 empiètent ainsi sur l'élément médian 110, soit au-delà de la ligne de pliage 150. Ces oreilles 134, 144 ont pour but de coopérer avec des découpes complémentaires sur le bord longitudinal 161 de l'élément externe 160 afin d'assurer le verrouillage en position de fermeture de l'emballage support 100.

La première partie 132 de l'élément interne 130, adjacente au bord latéral 101 comprend en outre un volet 136 articulé à pivotement. Le volet 136 est délimité par une découpe en U 137. Il est articulé sur la partie 132 à l'aide d'une ligne de pliage formée par une série de segments découpés rectilignes alignés et non jointifs 138. Les segments 138 raccordent les extrémités de la découpe en U 137.

Le volet 136 est positionné sur la partie 132 de façon à être situé en regard des fenêtres 113, 114 de l'élément médian 110 lorsque l'élément interne est placé contre l'élément médian 110 par pliage autour de la ligne 150. Le volet 136 a pour but d'éviter toute interférence entre la partie 132 de l'élément interne 130 et les broches placées dans les fenêtres 113, 114, tout en autorisant une immobilisation du fil bobiné.

Ainsi, comme on l'explicitera par la suite, pour l'assemblage de l'emballage support, l'élément interne 130 est plié contre l'élément médian 110 avant de retirer les broches placées dans les fenêtres 113, 114.

De préférence, la partie 132 est pourvue sur son bord longitudinal 131 et à proximité du bord latéral 101 d'une creusure 139 en retrait vers l'intérieur de la partie 131 suivi d'une dent 141 en saillie au-delà du bord longitudinal 131, laquelle dent 141 est adjacente au bord latéral 101.

Comme on le comprendra à l'examen des figures annexées, cette creusure 139 et cette dent 141 ont pour but de définir un maintien du fil dans sa zone adjacente à l'aiguille 22, tout en autorisant une certaine liberté au fil lors de l'ouverture de l'emballage.

On notera que le bord latéral 101 de l'élément interne 130 est situé en retrait de la découpe 115 formée dans l'élément médian 110 et débouchant sur le bord latéral 101 correspondant. En d'autres termes, la longueur de l'élément interne 130, considérée parallèlement au bord longitudinal 131, est inférieure à la longueur correspondante de l'élément médian 110.

La seconde partie 142 est pourvue d'une large découpe 146 qui relie le bord latéral 102 au bord longitudinal 131. Cette découpe 146 est placée en regard des fenêtres 111, 112 réalisées dans l'élément médian 110, pour permettre, de façon comparable au volet 136, le repli de la partie 142 contre l'élément médian 110, sans interférence de cette partie 142 avec les broches placées dans les fenêtres 111, 112.

On va maintenant décrire, la structure de l'élément externe 160 représentée sur les figures annexées.

Cet élément externe 160 comprend deux découpes rectilignes 161, 162 qui débouchent respectivement sur les bords latéraux 101, 102. Les découpes 161, 162 s'étendent perpendiculairement à ces bords latéraux 101, 102, à mi-largeur de l'élément externe 160. Ces découpes 161, 162 ont pour but de recevoir les deux formes triangulaires délimitées par les découpes 115, 116 dans l'élément médian 110, comme on le voit notamment sur la figure 1, pour assurer la fermeture de l'emballage en position fermée.

Le bord longitudinal 161 de l'élément externe 160, qui est opposé à la ligne de pliage 180 comprend des découpes conçues pour coopérer avec les pattes 134, 144 de l'élément interne 130. Plus précisément, il est prévu ainsi deux jeux de découpes 170, 175 conçus pour coopérer respectivement avec les pattes 134, 144.

Chaque jeu 170, 175 comprend deux découpes 171, 176 symétriques entre elles par rapport un plan orthogonal au bord longitudinal 161.

Plus précisément encore, chaque découpe 171, 176 comprend un premier segment 172, 177 et un second segment 173, 178.

Le premier segment 172, 177 est rectiligne et débouche sur le bord longitudinal 161 perpendiculairement à celui-ci. Le second segment 173, 178 prolonge le premier segment 172, 177 vers l'intérieur de l'élément externe 160. Le second segment 173, 178 rectiligne est incliné de quelques degrés par rapport au bord longitudinal 161. Il diverge par rapport à ce bord longitudinal 161 en rapprochement de son extrémité libre. Par ailleurs, les seconds segments 173, 178 de chaque jeu 170, 175 divergent entre eux en rapprochement de leurs extrémités libres.

La distance séparant les premiers segments 172 et 177 respectivement est inférieure à la largeur des pattes 134, 144 considérée parallèlement aux lignes de pliage 150, 180. Cependant, la distance séparant les extrémités des seconds segments 173 et 178 respectivement est supérieure à la largeur précitée des pattes 134, 144.

L'élément externe 160 comprend également un volet 163. Le volet 163 comprend une patte 164 en saillie au-delà de la ligne de pliage 180. La patte 164 est adjacente au bord latéral 101.

La patte 164 a pour but de faciliter la préhension du volet 163, afin de séparer celui-ci partiellement de l'élément externe 160 et de pivoter le volet 163, comme on l'évoquera par la suite.

Le volet 163 est délimité en partie par une ligne brisée formée de différents segments de découpe 165.

Le volet 163 est articulé sur l'élément externe 160 par une ligne constituée de différents segments de découpe 166, rectilignes, non jointifs et alignés entre eux. Ces segments 166 débouchent sur le bord latéral 101 à proximité du bord longitudinal 161. Les segments 166 s'étendent parallèlement à ce bord longitudinal 161.

Les segments 165 qui délimitent le volet 163, relient ainsi l'extrémité interne de la ligne de segments de découpe 166 à la ligne d'articulation 180, à proximité du bord latéral 101.

Le volet 163 est muni de plus de moyens permettant d'immobiliser de façon amovible l'aiguille 22 de la suture. Ces moyens d'immobilisation sont formés de préférence d'un bloc de mousse ou équivalent représenté sous la référence 167 sur la figure 4 annexée. Ce bloc de mousse 167, par exemple de contour rectangulaire, est collé de préférence sensiblement à mi-longueur du volet 163, à proximité de la ligne d'articulation à pivotement formée par les segments de découpe 166.

On notera que de préférence, les angles des trois éléments 110, 130 et 160 sont arrondis.

Pour assembler un emballage support de suture 100 conforme à la présente invention, on procède essentiellement comme suit.

Le flanc 100 est présenté à l'état plan, et après découpe des différentes structures précédemment décrites, sur une machine de bobinage classique. Des broches support de fil sont ainsi positionnées dans les fenêtres 111, 112, 113 et 114.

On procède de façon classique en soi au bobinage du fil de suture sur les broches précitées. Le bobinage du fil de suture est avantageusement réalisé en huit, de façon classique, pour éviter tout entrelacement intempestif du fil, à l'utilisation.

Les deux parties 132, 142 de l'élément interne 130 peuvent, après le bobinage, être repliées successivement sur l'élément médian 110, sans interférer avec les broches précitées. Le fil de suture est ainsi immobilisé correctement entre l'élément médian 110 et l'élément interne 130. Les broches précitées peuvent ensuite être retirées.

Les parties 132, 142 sont verrouillées sur l'élément médian 110 par introduction des saillies 133, 143 respectives dans la découpe centrale 183. L'aiguille 22 de la suture est piquée dans le bloc de mousse 167. L'élément externe 160 peut à son tour être replié contre l'élément interne 130.

L'élément externe 160 est immobilisé en engageant, d'une part les pattes 134, 144 dans les jeux de découpe 175, 170 et, d'autre part en engageant les formes définies par les découpes 115, 116 de l'élément médian 110 dans les découpes 162, 161.

A l'utilisation, il suffit de saisir la patte de manipulation 164 et de tirer à l'aide de celle-ci sur le volet 163, pour relier les segments 165 précédemment non jointifs et permettre ainsi le pivotement du volet 163 autour de la ligne d'articulation délimitée par les segments 166, comme on le voit sur les figures 2 et 3.

Le praticien peut alors accéder aisément et totalement à l'aiguille 22.

Il faut noter que selon une caractéristique importante de l'invention, l'aiguille 22 étant de préférence piquée dans le bloc de mousse 167 à distance de la liaison articulée 166, le pivotement du volet 163 par rapport à l'élément externe 160 entraîne un décollement de l'aiguille par rapport à l'emballage support, comme on le voit à l'examen de la figure 3. Ce décollement facilite la préhension ultérieure de l'aiguille 22, par exemple à l'aide d'un système de pince.

En outre, le volet 163 peut être utilisé pour guider le système de pince afin de faciliter cette préhension de l'aiguille 22.

L'emballe-support conforme à la présente invention est particulièrement simple, fiable, d'utilisation facile, et offre une parfaite immobilisation de l'aiguille 22 avant utilisation.

Le cas échéant l'emballage support 100, réalisé en carton, peut être imprimé sur sa face externe, afin d'identifier par exemple la nature du fil 20 ou de l'aiguille 22, et les dimensions de ceux-ci.

Pour cela, de préférence, l'enveloppe externe 10 comprend au moins une feuille optiquement transparente pour permettre de lire les informations imprimées sur l'emballage support 100, avant ouverture de l'enveloppe 10.

Bien entendu la présente invention n'est pas limitée au mode de réalisation particulier qui vient d'être décrit mais s'étend à toute variante conforme à son esprit.

Ainsi, selon une variante, l'emballage support 100 peut recevoir des sutures équipées de deux aiguilles 22 ou plus solidaires d'un même fil 20 ou de fils différents. Dans ce cas, il est avantageux de prévoir deux blocs de mousse ou plus sur l'élément externe 160. Ces deux blocs de mousse ou plus peuvent être fixés sur le volet articulé à pivotement 163 de sorte que l'ouverture de celui-ci assure un décollement des diverses aiguilles 22 simultanément.

Selon encore une autre variante on peut prévoir un bloc de mousse 167 recevant une première aiguille 22 fixée sur le volet 163, et au moins un second bloc de mousse destiné à recevoir la ou les autres aiguille(s) 22 collée(s) sur la face interne de l'élément 160 à l'extérieur du volet 163.

Eventuellement dans ce dernier cas, le ou les bloc(s) de mousse additionnel(s) peut(vent) être positionné(s) sur un ou des volet(s) addtionnel(s) similaire(s) au volet 163 articulé à pivotement sur l'élément externe 160 de sorte que les autres aiguilles puissent être à leur tour décollées de l'emballage support lors de l'ouverture du ou des volet(s) addtionnel(s) précité(s).

Comme on le comprend à l'examen de la figure 3, en position d'ouverture, le volet articulé 163 peut servir de jambe d'appui à l'emballage support permettant de maintenir celui-ci globalement vertical.

On notera également que selon l'invention, le fil de suture 20 est situé essentiellement entre l'élément médian 110 et l'élément interne 130, tandis que l'aiguille 22 est située entre l'élément interne 130 et l'élément externe 160. Ainsi, l'élément interne 130 permet d'isoler l'aiguille 22 du fil 20 ce qui évite toute détérioration du fil 20 au stockage.

## Revendications

1. Emballage-support de suture chirurgicale (20, 22), réalisé par découpes et pliages d'un flanc en carton comprenant trois éléments (110, 130, 160) séparés deux à deux par des lignes de pliage respectives (150, 180), le fil (20) de la suture étant situé essentiellement entre deux éléments adjacents (110, 130) de l'emballage support et l'emballage comprenant un volet d'ouverture (163) articulé à pivotement sur le troisième élément (160) caractérisé en ce que l'aiguille (22) de la suture est fixée sur ce volet d'ouverture (163) à distance de son axe d'articulation (166), et de la zone (139) d'émergence du fil (20) entre les deux éléments premiers cités, de sorte que lors de l'ouverture de l'emballage support, le pivotement du volet d'ouverture (163) provoque le décollement de l'aiguille (22) en éloignement de la structure de base de l'emballage support et facilite de ce fait la préhension ultérieure de l'aiguille (22).

2. Emballage-support selon la revendication 1, caractérisé par le fait que les moyens de fixation de l'aiguille (22) sur le volet (163) articulé à pivotement comprennent un bloc de mousse (167).

3. Emballage-support selon l'une des revendication 1 ou 2, caractérisé par le fait qu'il comprend au moins une ligne de pliage (180) conçue pour donner du volume à l'emballage support (100).

4. Emballage-support selon la revendication 3, caractérisé par le fait qu'il comprend au moins une ligne de pliage (180) comprenant deux séries de découpes (185, 188) sensiblement adjacentes et parallèles.

5. Emballage-support selon l'une des revendications 1 à 4, caractérisé par le fait qu'il comprend un élément médian (110) encadré par deux lignes de pliage (150, 180) et comprenant des fenêtres (111, 112, 113, 114) conçues pour le passage de broches servant d'appui au fil de suture lors du bobinage de celui-ci.

6. Emballage-support selon l'une des revendications 1 à 5, caractérisé par le fait qu'il comprend un élément (110) comportant des découpes (115, 116) qui débouchent sur des bords latéraux (101, 102) et sont inclinés sur ces derniers.

7. Emballage-support selon l'une des revendications 1 à 6, caractérisé par le fait qu'il comprend un élément (130) scindé en deux parties (132, 142) par une ligne de découpe (140).

8. Emballage-support selon la revendication 7, caractérisé par le fait que les deux parties (132, 142) comprennent respectivement une dent en saillie (133, 143) respectivement de part et d'autre de la ligne de découpe (140), lesquelles dents en saillie (133, 143) sont conçues pour pénétrer dans une découpe complémentaire (183) afin d'assurer un verrouillage de l'emballage support.

9. Emballage-support selon l'une des revendications 1 à 8, caractérisé par le fait qu'il comprend au moins un élément (130) comprenant au moins une patte en saillie (134, 144), conçue pour coopérer avec un jeu de découpes (170, 175) complémentaires afin d'assurer un verrouillage en position de fermeture de l'emballage support.

10. Emballage-support selon l'une des revendications 1 à 9, caractérisé par le fait qu'il comprend au moins un élément (130) comprennant un volet (136) articulé, situé après pliage en regard de fenêtres (113, 114) formées dans un autre élément de l'emballage support.

11. Emballage-support selon l'une des revendications 1 à 10, caractérisé par le fait qu'il comprend au moins un élément (142) comportant une découpe (146) située en regard de fenêtres (111, 112) placées dans un autre élément (110) après pliage de la structure de l'emballage support.

12. Emballage-support selon l'une des revendications 1 à 11, caractérisé par le fait qu'il comprend au moins un élément (160) comportant des découpes (161, 162) qui débouchent sur les bords latéraux (101, 102) de l'emballage et sont destinés à coopérer avec des découpes complémentaires (115, 116) formées dans un autre élément (110).

13. Emballage-support selon l'une des revendications 1 à 12, caractérisé par le fait qu'il comprend au moins un jeu de découpes (170, 175) conçu pour coopérer avec une patte en saillie (134, 144) formée sur un élément adjacent.

14. Emballage-support selon la revendication 13, caractérisé par le fait que le jeu de découpes (170, 175) comprend deux découpes (171, 176) comprenant chacune un premier segment (172, 177) qui débouche sur le bord longitudinal (161) de l'emballage support sensiblement orthogonalement à celui-ci et un second segment (173, 178) divergent vers l'intérieur de l'emballage.

15. Emballage-support selon l'une des revendications 1 à 14, caractérisé par le fait que le volet (163) articulé à pivotement comprend une patte de préhension (164) en saillie.

16. Emballage-support selon l'une des revendications 1 à 15, caractérisé par le fait qu'il comprend des informations imprimées sur sa surface extérieure.

17. Emballage-support selon l'une des revendications 1 à 16, caractérisé par le fait qu'il comprend au moins deux aiguilles (22).

18. Emballage-support selon la revendication 17, caractérisé par le fait qu'il comprend au moins deux blocs de mousse (167) conçus pour recevoir respectivement les aiguilles (22).

19. Conditionnement pour suture chirurgicale, caractérisé par le fait qu'il comprend une enveloppe externe stérile recevant un emballage support conforme à l'une des revendications 1 à 18.

## Claims

1. A support-wrapping for a surgical suture (20, 22), the support-wrapping comprising cuts and folds in a card blank that is made up of three elements (110, 130, 160) that are connected together in pairs by respective fold lines (150, 180), the thread (20) of the suture being essentially situated between two adjacent elements (110, 130) of the support-wrapping, and the wrapping further including a pivotally-hinged opening flap (163) on the third element (160), the support-wrapping being characterized in that the needle (22) of the suture is fixed to said opening flap (163) at a distance from its hinge axis (166) and also from the zone (139) where the thread (20) emerges from between the two first-mentioned elements, such that on opening the support-wrapping, the pivoting of the opening flap (163) causes the needle (22) to be moved away from the basic structure of the support-wrapping, thereby facilitating subsequent grasping of the needle (22).

2. A support-wrapping according to claim 1, characterized by the fact that the means for fixing the needle (22) on the pivotally-hinged flap (163) comprise a block of foam (167).

3. A support-wrapping according to claim 1 or 2, characterized by the fact that it includes at least one fold line (180) designed to give volume to the support-wrapping (100).

4. A support-wrapping according to claim 3, characterized by the fact that it includes at least one fold line (180) including two series of cuts (185, 188) that are substantially adjacent and parallel.

5. A support-wrapping according to any one of claims 1 to 4, characterized by the fact that it includes a middle element (110) between two fold lines (150, 180) and having windows (111, 112, 113, 114) designed to receive pegs that are used as supports for the suture thread while it is being coiled.

6. A support-wrapping according to any one of claims 1 to 5, characterized by the fact that it includes an element (110) that has cuts (115, 116) opening out into its end edges (101, 102) and that slope relative thereto.

7. A support-wrapping according to any one of claims 1 to 6, characterized by the fact that it includes an element (130) that is split into two portions (132, 142) by a line of cut (140).

8. A support-wrapping according to claim 7, characterized by the fact that both of the two portions (132, 142) comprise a respective projecting tongue (133, 143) on respective opposite sides of the line of cut (140), which projecting tongues (133, 143) are designed to penetrate into a complementary cut (183) so as to lock the support-wrapping.

9. A support-wrapping according to any one of claims 1 to 8, characterized by the fact that it includes at least one element (130) having at least one projecting lug (134, 144) designed to co-operate with a complementary set of cuts (170, 175) in order to lock the support-wrapping in a closed position.

10. A support-wrapping according to any one of claims 1 to 9, characterized by the fact that it includes at least one element (130) that has a hinged flap (136) which is situated, after folding, so as to overlie windows (113, 114) formed in another element of the support-wrapping.

11. A support-wrapping according to any one of claims 1 to 10, characterized by the fact that it includes at least one element (142) including a cutout (146) situated to overlie windows (111, 112) placed in another element (110) after the support-wrapping structure has been folded.

12. A support-wrapping according to any one of claims 1 to 11, characterized by the fact that it includes at least one element (160) including cuts (161, 162) that open out in the end edges (101, 102) of the wrapping and that are designed to co-operate with complementary cuts (115, 116) formed in another element (110).

13. A support-wrapping according to any one of claims 1 to 12, characterized by the fact that it includes at least one set of cuts (170, 175) designed to co-operate with a projecting lug (134, 144) formed on an adjacent element.

14. A support-wrapping according to claim 13, characterized in that each set of cuts (170, 175) comprises two cuts (171, 176) each having a first segment (172, 177) that opens out into the longitudinal edge (161) of the support-wrapping that is substantially orthogonal thereto, followed by a second segment (173, 178) diverging towards the inside of the wrapping.

15. A support-wrapping according to any one of claims 1 to 14, characterized by the fact that the pivotally hinged flap (163) includes a projecting tab (164) for grasping purposes.

16. A support-wrapping according to any one of claims 1 to 15, characterized by the fact that it includes information printed on its outside surface.

17. A support-wrapping according to any one of claims 1 to 16, characterized by the fact that it includes at least two needles (22).

18. A support-wrapping according to claim 17, characterized by the fact that it includes at least two foam blocks (167) designed to receive respective needles (22).

19. Packaging for a surgical suture, characterized by the fact that it comprises a sterile outer envelope receiving a support-wrapping according to any one of claims 1 to 18.

## Patentansprüche

1. Verpackungshilfe für chirurgisches Nahtmaterial (20, 22), gebildet durch Stanzen und Falten von einer aus Pappe bestehenden Seite, die drei Elemente (110, 130, 160) umfaßt, die paarweise durch entsprechende Linien von Falten (150, 180) getrennt sind, wobei der Faden (20) von dem Nahtmaterial sich im wesentlichen zwischen zwei angrenzenden Elementen (110, 130) der Verpackungshilfe befindet, die eine Öffnungsklappe (163) umfaßt, die schwenkbar an dem dritten Element (160) verbunden ist,
dadurch gekennzeichnet,
daß die Nadel (22) des Nahtmaterials an dieser Öffnungsklappe (163) in einer Entfernung von ihrer Drehachse (166) und von dem Bereich (139) des zum Vorschein Kommens des Fadens (20) zwischen den beiden zuerst genannten Elementen so befestigt ist, daß beim Öffnen der Verpackungshilfe das Schwenken der Öffnungsklappe (163) das Loslösen der Nadel (22) durch Entfernung der Basisstruktur der Verpackungshilfe auslöst und so das Ereignis des späteren Greifens der Nadel (22) erleichtert.

2. Verpackungshilfe nach Anspruch 1, dadurch gekennzeichnet, daß die Mittel zur Befestigung der Nadel (22) an der drehbar befestigten Klappe (163) einen aus Schaumstoff bestehenden Block (167) umfassen.

3. Verpackungshilfe nach Anspruch 1 oder 2, dadurch gekennzeichnet, daß es wenigstens eine Linie einer Falte (180) umfaßt, die vorgesehen ist, um der Verpackungshilfe ein Volumen zu verleihen.

4. Verpackungshilfe nach Anspruch 3, dadurch gekennzeichnet, daß es wenigstens eine Linie einer Falte (180) umfaßt, die zwei genau angrenzende und parallele Stanzreihen (185, 188) umfaßt.

5. Verpackungshilfe nach einem der Ansprüche 1 bis 4, dadurch gekennzeichnet, daß es ein mittleres Element (110) umfaßt, welches von zwei Faltlinien (150, 180) eingerahmt ist und welches Fenster (111, 112, 113, 114) umfaßt, die für den Durchgang von Stiften vorgesehen sind, um einen Halt für den Faden des Nahtmaterial als dessen Spule bereitzustellen.

6. Verpackungshilfe nach einem der Ansprüche 1 bis 5, dadurch gekennzeichnet, daß es ein Element (110) umfaßt, welches Schnitte (115, 116) aufweist, die an den seitlichen Rändern (101, 102) münden und schräg zu diesen verlaufen.

7. Verpackungshilfe nach einem der Ansprüche 1 bis 6, dadurch gekennzeichnet, daß es ein Element (130) umfaßt, welches in zwei Teile (132, 142) durch eine Linie eines Schnittvorgangs (140) getrennt ist.

8. Verpackungshilfe nach Anspruch 7, dadurch gekennzeichnet, daß die zwei Teile (132, 142) jeweils einen vorspringenden Zacken (133, 144) auf der jeweils einen und der anderen Seite der Linie des Schnittvorgangs (140) umfassen, wobei die vorspringenden Zacken vorgesehen sind, um in einen ergänzenden Schnitt (183) einzudringen, um eine Verriegelung zu gewährleisten.

9. Verpackungshilfe nach einem der Ansprüche 1 bis 8, dadurch gekennzeichnet, daß es wenigstens ein Element (130) umfaßt, das wenigstens eine vorstehende Lasche (134, 144) aufweist, die vorgesehen ist, um mit einer Garnitur von ergänzenden Schnitten (170, 175) zusammenzuwirken, um eine Verriegelung im geschlossenen Zustand der Verpackungshilfe zu gewährleisten.

10. Verpackungshilfe nach einem der Ansprüche 1 bis 9, dadurch gekennzeichnet, daß es wenigstens ein Element (130) umfaßt, welches eine befestigte Klappe (136) aufweist, die hinter der den Fenstern (113, 114) gegenüberliegenden Falte, in einem anderen Element der Verpackungshilfe liegend, gebildet ist.

11. Verpackungshilfe nach einem der Ansprüche 1 bis 10, dadurch gekennzeichnet, daß es Wenigstens ein Element (142) umfaßt, das einen Ausschnitt (146) aufweist, der den Fenstern (111, 112), die sich in einem anderen Element (110) hinter der Falte des Aufbaus der Verpackungshilfe befinden, gegenüberliegt.

12. Verpackungshilfe nach einem der Ansprüche 1 bis 11, dadurch gekennzeichnet, daß es wenigstens ein Element (160) umfaßt, das Schnitte (161, 162) enthält, die an den seitlichen Rändern (101, 102) der Verpackung münden und die dazu bestimmt sind, mit den ergänzenden Schnitten (115,116), die in einem anderen Element (110) gebildet sind, zusammenzuwirken.

13. Verpackungshilfe nach einem der Ansprüche 1 bis 12, dadurch gekennzeichnet, daß es wenigstens eine Garnitur von Schnitten (170, 175) umfaßt, die vorgesehen ist, um mit einer hervorstehenden Lasche (134, 144), die in einem benachbarten Element ausgebildet ist, zusammenzuwirken.

14. Verpackungshilfe nach Anspruch 13, dadurch gekennzeichnet, daß die Garnitur der Schnitte (170, 175) zwei Schnitte (171, 176) umfaßt, die jede einen ersten Abschnitt (172, 177) aufweisen, die an den Längsrand (161) der Verpackungshilfe genau senkrecht zu diesem münden, und einen zweiten Abschnitt (173, 178), der ins Innere der Verpackung verläuft.

15. Verpackungshilfe nach einem der Ansprüche 1 bis 14, dadurch gekennzeichnet, daß die schwenkbar befestigte Klappe (163) eine vorstehende Lasche zum Greifen aufweist.

16. Verpackungshilfe nach einem der Ansprüche 1 bis 15, dadurch gekennzeichnet, daß diese aufgedruckte Informationen auf der äußeren Oberfläche umfaßt.

17. Verpackungshilfe gemäß einem der Ansprüche 1 bis 16, dadurch gekennzeichnet, daß diese wenigstens zwei Nadeln (22) umfaßt.

18. Verpackungshilfe gemäß Anspruch 17, dadurch gekennzeichnet, daß diese wenigstens zwei Blöcke aus Schaumstoff (167) umfaßt, die für die entsprechenden Nadeln (22) vorgesehen sind.

19. Verpackung für chirurgisches Nahtmaterial, dadurch gekennzeichnet, daß diese einen äußeren sterilen Umschlag umfaßt, der eine Verpackungshilfe gemäß einem der Ansprüche 1 bis 18 enthält.
